# EUROPEAN PATENT APPLICATION

(11) **EP 1 723 951 A1**
(43) Date of publication of application: **22.11.2006**
(21) Application number: 05103257.1
(22) Date of filing: 21.04.2005
(51) Int. Cl.: A61K 31/00

(54) **Nutritional supplement with oligosaccharides for a category of HIV patients**

(71) Applicant: N.V. Nutricia, 2712 HM Zoetermeer (NL)
(72) Inventor: Garssen, Johan, 3433 DA Nieuwegein (NL); Van Tol, Eric Alexander Franciscus, 6824 MZ Arnhem (NL); Sijben, Johannes Wilhelmus Christina, 6701 BA Wageningen (NL); Verlaan, George, 6708 SB Wageningen (NL)
(74) Representative: van Westenbrugge, Andries

(57) **Abstract**

The present invention relates to a nutritional product for HIV patients. More specifically the invention relates to a nutritional composition that provides carefully selected nutritional ingredients including oligosaccharides and cysteine and/or a source of cysteine specifically supporting HIV patients with nutritionally related symptoms. This invention also relates to the manufacture of a nutritional supplement for use in HIV patients.

## Description

### FIELD OF THE INVENTION

The present invention relates to a nutritional product for HIV patients. More specifically the invention relates to a nutritional composition that provides carefully selected nutritional ingredients specifically supporting HIV patients with nutritionally related symptoms. This invention also relates to the manufacture of a nutritional supplement for use in HIV patients.

### BACKGROUND OF THE INVENTION

Infections with the human immunodeficiency virus (HIV) and the development of acquired immunodeficiency syndrome (AIDS) have had a significant impact on domestic and global health, social, political, and economic outcomes. Worldwide, the number of HIV-1 infected persons exceeds 40 million, the majority of whom live in Asia, sub-Saharan Africa and South America. Despite all the therapeutic advantages achieved during the last decade, including the development of highly active antiretroviral therapy ("HAART"), once an individual has become infected, eradication of the virus still remains impossible.

The importance of nutritional support of HIV infected persons is recognized nowadays. Infected patients may have increased needs for basal energy, proteins, and micronutrients due to the metabolic stress they experience. This stress, coupled with the anorexia and malabsorption associated with the disease, promotes malnutrition. Malnutrition generally affects e.g. the immune-competence, (work) performance and cognition. Providing extra nutrition helps these patients to improve their general nutritional status.

Currently several products are on the market for nutritional support of HIV patients. Different commercial suppliers have several clinical nutrition products on the market, which are listed below.

### 1. Advera, Ross Abbott

Caloric Distribution:
Protein: 18.7% (Soy protein hydrolysate, Sodium Caseinate)
Carbohydrate: 65.5% (maltodextrin, sucrose, soy fiber)
Fat: 15.8% (Canola, MCT, Refined, deodorized sardine oil 1.5 e%)
Caloric Density: 1.28 kcal/mL

### 2. Resource, Novartis

Caloric Distribution:
Protein: 14% (Sodium and Calcium Caseinates, Soy Protein Isolate)
Carbohydrate: 64% (Corn Syrup, Sugar)
Fat: 22% (High Oleic Sunflower Oil, Corn Oil)
Caloric Density: 1.06 kcal/mL

### 3. Benecalorie, Novartis

Caloric Distribution:
Protein: 9% (Calcium Caseinate)
Carbohydrate: 0%
Fat: 91 % (High Oleic Sunflower Oil, Mono and Diglycerides)
Caloric Density: 7 kcal/mL

### 4. Boost, Mead Johnson now product sold by Novartis

Caloric Distribution:
Protein: 24% (milk protein concentrate, Ca & Na caseinates)
Carbohydrate: 55% (corn syrup solids, sugar)
Fat: 21% (canola, high oleic sunflower and corn oils)
Caloric Density: 1.01 kcal/mL

However, despite the availability of products which support the general nutritional requirements of HIV infected patients, there are no nutritional products available which do not only improve the nutritional status but which additionally significantly reduce or prevent specific HIV infection related symptoms, in particular immune dysfunction, intestinal dysfunction and/or glutathione status of the subjects.

### SUMMARY OF THE INVENTION

The current nutritional treatments of HIV patients have the disadvantage that these do not give an overall solution for all the nutritionally related medical problems of HIV patients, in particular infection related immune dysfunction, intestinal dysfunction and/or glutathione status. In one embodiment the present invention relates to the use of oligosaccharides and cysteine and/or source of cysteine in the manufacture of a composition for use in a method for the treatment and/or prevention of HIV or AIDS, said method comprising administering to a mammal a composition comprising a therapeutically effective amount of oligosaccharide and cysteine and/or source of cysteine. In another embodiment the compositions further comprise one or more polyunsaturated fatty acids (PUFAs) and/or one or more biologically active compounds, in particular milk-derived compounds.

The present invention provides complete nutritional supplements suitable for the nutritional treatment of HIV patients. The nutritional supplements of the present invention comprise at least 2, preferably at least 3 components supporting the subject's gut function, glutathione (GSH) status and/or immune function. It was surprisingly found that, by carefully choosing combinations of nutritional ingredients, several nutrition-related side effects of the HIV infection (i.e. infection related symptoms) can be prevented and/or significantly reduced. The effect was found to be much better when several disease related symptoms were targeted at the same time than when the patient was given only one of the individual ingredients as has been practiced until today.

A healthy gut and healthy gut flora are intricately linked to healthy immune function. Potential immune modulating effects by specific fibers/oligosaccharides may be the indirect result of the influence on the gut flora composition (immune effects of bifidobacteria and lactobacilli types have been documented) and/or function (fermentation of fibers produces compounds such as short chain fatty acids that influence general and immunological function of gut cells). Surprisingly the inventors found that the DC-SIGN molecule of dendritic cells can be blocked by certain oligosaccharides. As the blockage of this molecule can potentially prevent the transmission of HIV, the use of these oligosaccharides for blocking the DC-SIGN receptor and for the manufacture of compositions for the prophylaxis and/or treatment of DC-SIGN mediated diseases (in particular HIV and AIDS) is provided in one embodiment of the invention.

### DETAILED DESCRIPTION

### General definitions

"Oligosaccharides" refers to carbohydrate chains of monosaccharide units with a chain length of between 1 and 5000, more preferably between 2 and 250, more preferably between 2 and 50, most preferably between 2 and 10.

"Degree of polymerization" or "DP" refers to the total number of saccharide units in an oligosaccharide chain. The "average DP" refers to the average DP of oligosaccharide chains in a composition, without taking possible mono- or disaccharides into account (which are preferably removed if present). The average DP of a composition is used to distinguish between compositions. Preferably the average degree of polymerization of oligosaccharide mixtures is between 2 and 100, more preferably between 3 and 250, e.g. between 3 and 50.
"Co-administration" of two or more substances refers to the administration of these substances to one individual, either in one composition or in separate compositions (kit of parts; as a combined composition), which are administered at the same time (simultaneously) or within a short time-span (separate or sequential use, e.g. within minutes or hours).
The term "comprising" is to be interpreted as specifying the presence of the stated parts, steps or components, but does not exclude the presence of one or more additional parts, steps or components.
"Percentage" or "average" generally refers to percentages of averages by weight, unless otherwise specified or unless it is clear that another basis is meant.
"GOS" or "galactooligosaccharides", or "trans-galactooligosaccharides" or "TOS" refers to oligosaccharides composed of galactose units.
"Treatment and/or prevention of HIV" refers to the significant reduction or prevention of one or more of HIV infection related symptoms/dysfunctions selected from immune dysfunction, intestinal dysfunction and/or low glutathione status. In one embodiment treatment or prevention of HIV refers to a significant reduction in (or a complete prevention of) the spread of HIV due to blockage of the DC-SIGN receptor, as will be clear from the context.
A "significant reduction or prevention" refers to a reduction of the symptom (or spread of HIV) by at least 5%, 10%, 15%, 30%, 50% or even 100% compared to control subjects, not being administered the compositions according to the invention. The symptoms can be measured as known in the art, e.g. immune dysfunction can be assessed by measuring CD4⁺ cell counts. Blockage of the DC-SIGN receptor can be determined as in Example 1.

The object of the present invention is to provide nutritional compositions suitable for treating HIV patients in order to improve their nutritional status and at least two, preferably at least three HIV related symptoms. The compositions according to the invention are particularly useful for patients with a CD4⁺ T cell count that is below the critical level of around 700 cells/µl blood, when generally HAART therapy is not yet needed, but when patients do already develop or experience one or more of the immune-, intestinal- and/or glutathione related dysfunctions.

Thus, the present compositions are suitable for prevention and/or treatment of one or more of HIV infection related dysfunctions, in particular:
1. immune dysfunction, i.e. a decrease in CD4⁺ T cell count leading to impaired immune function;
2. intestinal dysfunction, i.e. intestinal problems, specifically HIV induced malabsorption and diarrhea; and/or
3. low glutathione status, specifically low glutathione levels in the blood and intracellularly in the T cells.

In a preferred embodiment the compositions are suitable for treatment or prevention of at least immune dysfunction and low glutathione status. These compositions comprise suitable amounts of both oligosaccharides and cysteine and/or source of cysteine. In another embodiment the compositions further comprise suitable amounts of one or more PUFA(s) and/or one or more biologically active compounds and are suitable for treatment or prevention of all three of the above dysfunctions.

Since CD4⁺ T-lymphocytes are infected and destroyed by HIV, the progression of HIV can be routinely and regularly monitored by measuring the CD4⁺ T-lymphocyte count in the circulation. The initial period after infection with HIV, which can last from three to more than ten years, is characterized by a slow but gradual decline in total CD4⁺ T-cell counts, with no apparent symptoms of decreased resistance to infections. The first signs of infectious complications usually occur when CD4⁺ T cell counts are below 700 cells /µl blood. At this point, the HIV seropositive individual may experience respiratory (coughs, colds, flu) and/or gastrointestinal (bowel discomfort, diarrhea) symptoms. These symptoms are still relatively mild and may be considered sub clinical; although bothersome to the individual, they are usually not sufficiently severe to cause hospitalization or the initiation of highly active antiretroviral treatment (HAART).

One of the cell types first encountered by human immunodeficiency virus type 1 (HIV-1) following sexual transmission is dendritic cells (DC). DC capture HIV-1 through C-type lectin receptors, of which the best-studied example is DC-SIGN, which mediates HIV-1 internalization. DC can keep the virus infectious for several days and are able to transmit HIV-1 to CD4(+) T cells. As is described in Example 1, the present inventors surprisingly found that oligosaccharides can bind to DC-SIGN.

### Compositions and uses according to the invention

The compositions according to the invention are suitable for the treatment and/or prevention of HIV and/or AIDS in a mammalian subject. The subjects are preferably human subjects infected with HIV and comprising a CD4⁺ cell count of about 700 cell per µl blood or less, more preferably between about 200 and 700 cells per µl, e.g. between about 200 and 500 cells or between about 200 and 600 or 500 and 700 cells per µl blood. In one embodiment the subjects have a CD4+ cell count of 700 or less but are not on highly active antiretroviral therapy (HAART),
In one embodiment the nutritional compositions are preferably food supplements and comprise oligosaccharides and cysteine and/or source of cysteine.

### Oligosaccharides

The compositions according to the invention comprise a therapeutically effective amount of oligosaccharides, preferably acid oligosaccharides and/or neutral oligosaccharides as described below.

Acid oligosaccharides comprise at least one acidic group while neutral oligosaccharides do not have such an acidic group. Dietary fibers have been extensively investigated for their health-beneficial effects. Some fibers are insoluble and non-fermentable and pass unchanged through the gut. Other fiber types may serve as prebiotics, i.e., they are used by gut bacteria and stimulate their growth. Thus, fibers such as inulin or oligosaccharides such as galactooligosaccharides (GOS) and fructo-oligosaccharides (FOS) have been documented to stimulate growth of bifidobacteria and lactic acid bacteria, which are important for a healthy gut flora.

### Acid oligosaccharides

The term "acid oligosaccharide(s)" refers to oligosaccharides comprising at least one acidic group selected from the group consisting of N-acetylneuraminic acid, N-glycoloylneuraminic acid, free or esterified carboxylic acid, sulfuric acid group and phosphoric acid group. In one embodiment the acid oligosaccharide preferably is a polyhexose. Preferably, at least one of the aforementioned acid groups is situated at the terminal hexose unit of the acid oligosaccharide. Preferably the acid oligosaccharide has the structure as depicted in Fig.1, wherein the terminal hexose (left) preferably comprises a double bond. Preferably the acid oligosaccharide contains a carboxylic acid at the terminal hexose unit, wherein said carboxylic acid group may be free or esterified. Methods for the manufacture of esterified pectin hydrolysates that can be suitably used in the present method and composition are provided in WO 01/60378 and/or WO 02/42484, which are hereby incorporated by reference. The hexose units other than the terminal hexose unit(s) are preferably uronic acid units, even more preferably galacturonic acid units. The carboxylic acid groups on these units may be free or (partly) esterified, and preferably at least 10% is methylated (see below).

### Fig.1. Polymeric acid oligosaccharide

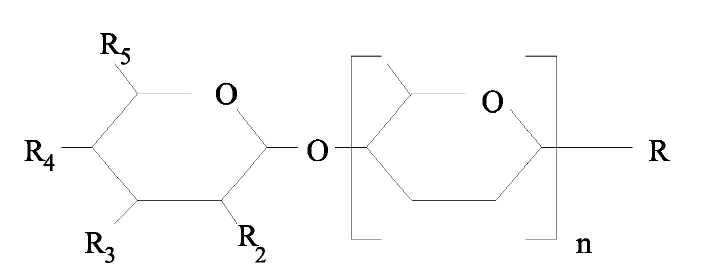

wherein:
R is preferably selected from the group consisting of hydrogen, hydroxy or acid group, preferably hydroxy; and
at least one selected from the group consisting of R₂, R₃, R₄ and R₅ represents N-acetylneuraminic acid, N-glycoloylneuraminic acid, free or esterified carboxylic acid, sulfuric acid group and phosphoric acid group, and the remaining of R₂, R₃, R₄ and R₅ representing hydroxy and/or hydrogen. Preferably one selected from the group consisting of R₂, R₃, R₄ and R₅ represents N-acetylneuraminic acid, N-glycoloylneuraminic acid, free or esterified carboxylic acid, sulfuric acid group or phosphoric acid group, and the remaining represent hydroxy and/or hydrogen. Even more preferably one selected from the group consisting of R₂, R₃, R₄ and R₅ represents free or esterified carboxylic acid and the remaining of R₂, R₃, R₄ and R₅ representing hydroxy and/or hydrogen; and
n is an integer and refers to a number of hexose units (see also Degree of Polymerisation, below), which may be any hexose unit. Suitably n is an integer between 1-5000. Preferably the hexose unit(s) is an uronic acid unit.
Most preferably R₁, R₂ and R₃ represent hydroxy, R₄ represent hydrogen, R₅ represents carboxylic acid, n is any number between 1 and 250, preferably between 1 and 10 and the hexose unit is galacturonic acid.

The detection, measurement and analysis of the acid oligosaccharides as used in the present method are given in applicant's earlier patent application relating to acid oligosaccharides, i.e. WO 01/60378, which is hereby incorporated by reference.

Preferably, the acid oligosaccharide has one, preferably two, terminal uronic acid units, which may be free or esterified. Preferably the terminal uronic acid unit is selected from the group consisting of galacturonic acid, glucuronic acid, guluronic acid, iduronic acid, mannuronic acid, riburonic acid and alturonic acid. These units may be free or esterified. In one embodiment, the terminal hexose unit has a double bond, which is preferably situated between the C₄ and C₅ position of the terminal hexose unit. Preferably one of the terminal hexose units comprises the double bond. The terminal hexose (e.g. uronic acid) preferably has a structure according to Fig.2.

### Fig. 2: Preferred terminal hexose acid group

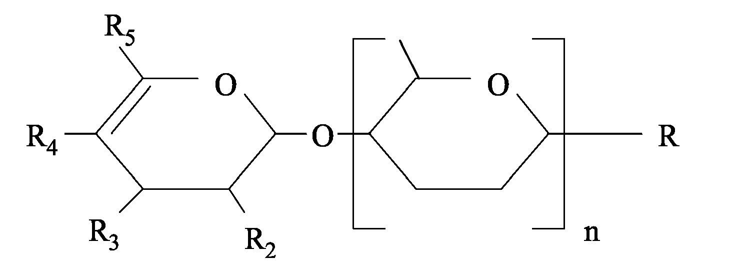

wherein;
R is preferably selected from the group consisting of hydrogen, hydroxy or acid group, preferably hydroxy (see above); and
at least one selected from the group consisting of R₂, R₃, R₄ and R₅ represents N-acetylneuraminic acid, N-glycoloylneuraminic acid, free or esterified carboxylic acid, sulfuric acid group and phosphoric acid group, and the remaining of R₂, R₃, R₄ and R₅ representing hydroxy and/or hydrogen. Preferably one selected from the group consisting of R₂, R₃, R₄ and R₅ represents N-acetylneuraminic acid, N-glycoloylneuraminic acid, free or esterified carboxylic acid, sulfuric acid group and phosphoric acid group, and the remaining of R₂, R₃, R₄ and R₅ represent hydroxy and/or hydrogen. Even more preferably one selected from the group consisting of R₂, R₃, R₄ and R₅ represents free or esterified carboxylic acid and the remaining of R₂, R₃, R₄ and R₅ represent hydroxy and/or hydrogen; and n is an integer and refers to a number of hexose units (see also Degree of Polymerisation, below), which may be any hexose unit. Suitably n is an integer between 1-5000 representing the number of hexose units, said hexose units preferably being uronic acid, even more preferably being galacturonic acid units. The carboxylic acid groups on these units may be free or (partly) esterified, and are preferably at least partly methylated.
Most preferably, R₂ and R₃ represent hydroxy, R₄ represent hydrogen and R₅ represents free or esterified carboxylic acid.

In one embodiment the compositions comprise a single type of acid oligosaccharide (having a uniform degree of polymerization), while in another embodiment the compositions comprise a mixture of acid oligosaccharides that have different Degrees of Polymerization (DP) and/or comprise both unsaturated and saturated terminal hexose unit. Preferably at least 5%, more preferably at least 10%, even more preferably at least 25% of the terminal hexose units of the acid oligosaccharide unsaturated hexose unit (see e.g. Fig.2). As each individual acid oligosaccharide preferably comprises only one unsaturated terminal hexose unit, preferably no more than 50% of the terminal hexose units is an unsaturated hexose unit (i.e. comprises a double bond).
A mixture of acid oligosaccharides preferably contains between 2 and 50% unsaturated hexose units based on the total amount of hexose units, preferably between 10 and 40%.

The acid oligosaccharide as used in the present method has a degree of polymerisation (DP) between 1 and 5000, preferably between 1 and 1000, more preferably between 2 and 250, even more preferably between 2 and 50, most preferably between 2 and 10. If a mixture of acid oligosaccharides with different degrees of polymerisation is used, the average DP of the acid oligosaccharide mixture is preferably between 2 and 1000, more preferably between 3 and 250, even more preferably between 3 and 50. See also Fig.1, wherein the sum of "n" and the terminal unit (i.e. n+1) represents the degree of polymerisation. It was found that a lower DP of the oligosaccharides improves the palatability and results in a reduced viscosity product if the acid oligosaccharide is administered in liquid form. The acid oligosaccharide may be a homogeneous or heterogeneous carbohydrate.

The acid oligosaccharides used in the invention are preferably prepared from pectin, pectate, alginate, chondroitine, hyaluronic acids, heparine, heparane, bacterial carbohydrates, sialoglycans, fucoidan, fucooligosaccharides or carrageenan, preferably from pectin and/or alginate. The acid oligosaccharides may be prepared by the methods described in WO 01/60378, e.g. chemical or enzymatic hydrolysis or partial hydrolysis, see page 8 and 9, which is hereby incorporated by reference.

Alginates are linear unbranched polymers containing β-(1→ 4)-linked D-mannuronic acid and α-(1→ 4)-linked L-guluronic acid residues with a wide range of average molecular weights (100 - 100000 residues). Suitable sources of alginate include seaweeds and bacterial alginates.

Pectin is divided into two main categories: high methoxylated pectin, which is characterised by a degree of methoxylation above 50% and low methoxylated pectin having a degree of methoxylation below 50%. As used herein, "degree of methoxylation" (also referred to as DE or "degree of esterification") is intended to mean the extent to which free carboxylic acid groups contained in the polygalacturonic acid chain have been esterified (e.g. by methylation). The present acid oligosaccharide is preferably prepared from high methoxylated pectin.

The acid oligosaccharides are preferably characterised by a degree of methoxylation above 20%, preferably above 50 % even more preferably above 70%. Preferably the acid oligosaccharides have a degree of methylation above 20%, preferably above 50 % even more preferably above 70%.

The acid oligosaccharide(s) is/are preferably administered in an amount of between about 10 mg and 100 gram per day, preferably between about 100 mg and 50 grams per day, even more preferably between about 0.5 and 20 gram per day.

### Neutral oligosaccharides

As mentioned above, the compositions may also comprise one or more neutral oligosaccharides, either instead of or in addition to one or more acid oligosaccharides. One or more neutral oligosaccharides are selected from the group consisting of cellobiose, cellodextrins, B-cyclodextrins, indigestible dextrin, gentiooligosaccharides, glucooligosaccharides, isomaltooligosaccharides, isomaltose, isomaltriose, panose, leucrose, palatinose, theanderose, D-agatose, D-*lyxo*-hexulose, lactosucrose, α-galactooligosaccharides, β-galactooligosaccharides, transgalactooligosaccharides, lactulose, 4'-galatosyllactose, synthetic galactooligosaccharide, fructans - Levan-type, fructans - Inulin-type, 1 f-β-fructofuranosylnystose, lacto N-tetraose, lacto N-neotetraose, xylooligosaccharide, lafmose, lactosucrose and arabinooligosaccharides.

Preferably the neutral oligosaccharide is selected from the group consisting of galactooligosaccharide, fructooligosaccharide, transgalactooligosaccharide xylooligosaccharide, lactosucrose and arabinooligosaccharides. Even more preferably the neutral oligosaccharide is selected from the group consisting of galactooligosaccharide, fructooligosaccharide and transgalactooligosaccharide.

Preferably the composition comprises two chemically distinct neutral oligosaccharides, one selected from the group consisting of galactose based neutral oligosaccharide and one selected from the group of fructose and/or glucose based oligosaccharide.

More preferably the composition comprises fructooligosaccharide and at least one oligosaccharide selected from transgalactooligosaccharride and galactooligosaccharide.

Preferred daily amounts of neutral oligosaccharides are between about 10 mg and 100 gram per day, preferably between about 100 mg and 50 grams per day, even more preferably between about 0.5 and 20 gram per day.

Preferably a composition comprising neutral and acid oligosaccharides is used wherein at least 15% of the total oligosaccharides comprise of acid oligosaccharides more preferably between 10 and 90% and most preferably between 25 and 75%. Preferably a composition is used wherein at least 25% of the oligosaccharides are acid oligosaccharides comprising at least one terminal uronic acid unit.

### Cysteine or source of cysteine

The compositions provided comprise in addition to one or more oligosaccharides as described above a suitable amount of cysteine and/or source of cysteine. The phrase "source of cysteine" refers herein to all compounds that contain a biologically available cysteine, in any form, and is calculated as the amount of cysteine amino acid that is present in a compound, or can be derived from a compound in the body after ingestion, on a molar basis.

Hereinbelow "cysteine equivalent" refers to an amount of cysteine as such or to an amount of cysteine that is present in a source of cysteine. For example 100 mg NAC (N-acetylcysteine; MW= 163.2) is equivalent to 74 mg cysteine (MW 121.15). Thus 100 mg NAC is 74 mg cysteine equivalent. Similarly this can be applied to proteins or peptides. When a peptide (MW = xDalton) contains 3 cysteine amino acids (3yDalton), than 100 mg of this peptide is equivalent to 100x3Y/X mg cysteine. Thus 100mg of this peptide is 300y/x mg cysteine equivalent.

Suitable sources of cysteine according to the invention are, for example, proteins in denatured and/or undenatured form such as milk proteins e.g. whey or casein proteins. Egg proteins are rich in cysteine and are therefore also suitable. Plant proteins such as pea, potato, soy and rice can also be used to provide cysteine. Also hydrolysates of these protein sources can be used or fractions enriched for cysteine rich proteins or peptides (e.g. as described in EP1201137). Furthermore, synthetic cysteine equivalents, e.g. derivatives of cysteine, such as cysteine, cysteine salts, N-acetylcysteine and/or diacetylcysteine can be used.

The HIV infected subjects are suitably administered a daily dose of at least about 100 mg cysteine equivalent, preferably at least about 200, 400, or 600 mg cysteine equivalent per day, more preferably at least about 1000 mg cysteine equivalent per day. It is understood that a daily dosage can be subdivided into 2, 3 or more dosage units taken several times a day.

In yet another embodiment the compositions according to the invention comprise one or more compounds that stimulate glutathione levels. e.g. lipoic acid, pyruvate, oxaloacetate, oxaloaspartate, are capable in stimulating glutathione levels. Such glutathione level stimulating compounds may be used in addition to cysteine but also instead of cysteine.

In another embodiment the compositions comprising one or more oligosaccharides and cysteine and/or source of cysteine further comprise one or more PUFAs and/or one or more biologically active compounds, such as compounds found in milk or probiotic microorganisms.

### Probiotic micro-organism

Probiotic micro-organism means a micro-organism which beneficially affects a HIV patient by improving its intestinal microbial balance (Fuller, R. J. Applied Bacteriology, 1989;66:365-378). The probiotic micro-organism may be selected from one or more microorganisms suitable for human consumption and which is able to improve the microbial balance in the intestine. Preferably, the present composition contains 10⁴ to 10¹², more preferably from 10⁵ to 10¹¹, most preferably from 10⁷ to 5x10¹⁰ colony forming units (cfu) of probiotic bacteria per gram uronic acid oligosaccharide with a DP between 2 and 100. The present composition preferably contains 10² to 10¹³ colony forming units (cfu) of probiotic bacteria per gram dry weight of the present composition, preferably 10⁴ to 10¹², more preferably 10⁵ to 10¹⁰, most preferably from 10⁵ to 1x10⁹ cfu. The dosage of probiotic bacteria according to the present invention is preferably between 10² to 10¹³, more preferably from 10⁵ to 10¹¹, most preferably from 10⁸ to 5x10¹⁰ colony forming units (cfu) per day. Preferably live or viable bacteria are used, but dead bacteria or bacterial fragments may also be used.

Preferably the present composition comprises bacteria of the genus Lactobacillus and/or Bifidobacterium. Preferably the composition comprises a Bifidobacterium selected from the group consisting of *B. longum, B.breve* and *B. bifidum* and/or a Lactobacillus selected from the group consisting of *L. casei, L paracasei, L. rhamnosus, L. acidophilus* and *L. plantarum*. Most preferably the present composition comprises *Bifidobacterium breve* and/or *Lactobacillus paracasei*.

*Bifidobacterium breve* is a Gram-positive, anaerobic, rod-shaped bacterium. The present *B. breve* preferably has at least 95 % nucleic acid sequence identity of the 16 S rRNA sequence when compared to the type strain of *B. breve* ATCC 15700, more preferably at least 97%, 98%, 99% or more sequence identity as defined in Stackebrandt & Goebel, 1994, Int. J. Syst. Bacteriol. 44:846-849. Nucleic acid sequence identity is calculated for two nucleotide sequences, when optimally aligned, using the programs GAP or BESTFIT using default parameters. The GAP default parameters are used, with a gap creation penalty = 50 (nucleotides) / 8 (proteins) and gap extension penalty = 3 (nucleotides) / 2 (proteins). For nucleotides the default scoring matrix used is nwsgapdna (Henikoff & Henikoff, 1992, PNAS 89, 915-919). It is clear than when RNA sequences are said to be essentially similar or have a certain degree of sequence identity with DNA sequences, thymine (T) in the DNA sequence is considered equal to uracil (U) in the RNA sequence. Sequence alignments and scores for percentage sequence identity may be determined using computer programs, such as the GCG Wisconsin Package, Version 10.3, available from Accelrys Inc., 9685 Scranton Road, San Diego, CA 92121-3752, USA or EMBOSSwin v. 2.10.0. The *Bifidobacterium* used in the present invention preferably hybridises with the *B. breve* probe and gives a signal with the 5' nuclease assay method as described in co-pending international patent application PCT/NL2004/000748 and european patent application 05075486.0 of the present applicant. According to a preferred embodiment, the present composition contains at least one *B. breve* selected from the group consisting of *B. breve* Bb-03 (Rhodia), *B. breve* M16-V (Morinaga), *B. breve* R0070 (Institute Rosell, Lallemand), DSM 20091, and LMG 11613. Most preferably, the *B. breve* is *B. breve* M-16V (Morinaga).
In a preferred embodiment the present composition comprises *Lactobacillus paracasei*. Preferably the present *L. paracasei* strain has at least 95%, more preferably at least 97%, 98%, 99% or more nucleic acid sequence identity of the 16S rRNA sequence when compared to the type strain of *L. paracasei* ATCC 25032 as defmed above. The *Lactobacillus* used in the present invention preferably hybridises with the *L. paracasei* probe and gives a signal with the 5' nuclease assay method as described in co-pending european patent application 05075486.0 of the present applicant. According to a preferred embodiment, the present composition contains at least a *L. paracasei* selected from the group consisting of *L. paracasei* F19 (Arla, Sweden), *L. paracasei* LAFTI L26 (DSM Food Specialties, the Netherlands) and *L. paracasei* CRL 431 (Chr. Hansen, Denmark), LMG 12165 and LMG 11407.

### Polyunsaturated fatty acids:

The present inventors found that eicosapentaenoic acid (EPA, n-3) and gamma linolenic acid (GLA, n-6) effectively reduce inflammatory mediated intestinal tight junction permeability. Hence a composition, suitable for improving intestinal barrier integrity is provided, which comprises (in addition to oligosaccharides and cysteine and/or source of cysteine) EPA and/or GLA. Based on the biochemical pathways it can be hypothesized that also other combinations of fatty acids are also effective. Thus, compositions comprising one or more other PUFAs or mixtures thereof are also provided. For example a mixture of any of EPA, docosahexaenoic acid (DHA, n-3), dihomo-gamma linolenic acid (DGLA, C20:3n-6), stearidonic acid (STA, C18:4n-4), alpha linolenic acid (ALA, C18:3n-3), (docosapentaenoic acid (DPA, C22:5n-3), eicosatetranoic acid (C20:4n-3) and/or arachidonic acid (AA, n-6) may be used.

Suitably a relatively high daily dose of the polyunsaturated fatty acids is used. In one embodiment at least about 25 en%, preferably at least about 30 en%, more preferably at least about 35 en% of a fat blend comprising n-3 and/or n-6 fatty acids is used (en% is short for energy percentage and represents the relative amount each constituent contributes to the total caloric value of the preparation). Preferred daily amounts are at least 1 gram PUFA, more preferably between 1-50 gram PUFA, more preferably between 5 and 25 gram PUFA and most preferred is an amount between 7.5 and 15 gram PUFA.

An optimal fat blend may e.g. comprise 40% borage oil and 60% fish oil. The n-3/n-6 fatty acid ratio is then between 1-2 and the weight percentage of n-3 is between 20-40, and of n-6 is between 15-35 of total fatty acid content. Borage oil can partly or completely be replaced by evening primrose oil.

Therefore preferred daily amounts are at least 0.1 gram EPA and 0.05 gram GLA, more preferably between 0.1 and 5 gram EPA and between 0.05 and 2.5 gram GLA, more preferably between 0.5 and 2.5 gram EPA and between 0.25 and 1.25 gram GLA and most preferred is an amount between 0.75 and 1.5 gram EPA and between 0.37 and 0.75 gram GLA.

### Biologically active ingredients

The compositions according to the invention may further comprise one or more biologically active molecules, preferably components found naturally in milk. These include growth factors, immunoglobulins, and other milk components or milk derived components.

### A. Growth factors

It has been found that milk growth factors are beneficial for gut health. Transforming growth factor-beta, insulin like growth factor and keratinocyte growth factors are the most important examples of milk growth factors. Therefore, in one embodiment the compositions further comprise one or more growth factors, e.g. about 1-500 µg growth factors per day.

### B. Immunoglobulins

Immunoglobulins have been shown to protect against intestinal infections and the compositions according to the invention suitably comprise a daily dose from 0,1 to 10g Immunoglobulins

### C. Other ingredients

Other bioactive ingredients obtainable from milk e.g. nucleotides, fatty acids, oligosaccharides were also found to have a beneficial effect on the gut barrier function and may therefore be suitably used in the manufacture of the compositions.

### D. Colostrum

In one embodiment the compositions comprise Colostrum. Colostrum is the pre-milk fluid secreted by the mammary glands of mammalian mothers after giving birth, in particular cows after calving. Colostrum contains many biologically active milk ingredients and is therefore an excellent source of biologically active molecules. Colostrum, being a protein source, has the additional advantage of providing cysteine. For having beneficial effects in HIV patients at least about 5 gram colostrum are provided on a daily basis, preferably at least about 10 gram, more preferably at least about 20 g per day or more.

Extracts from milk proteins, such as a whey growth factor extract as described in EP0545946 or a casein extract as described in WO02083164, immunoglobulin concentrates, lactoferrin or other concentrated whey fractions can also be used to improve the gut barrier function of HIV patients.

It is understood that the biologically active molecules or components may be obtained using a range of methods. Many are commercially available, or can be made synthetically, by recombinant DNA technology or they can be (partially) purified or extracted from natural sources such as milk. Also mixtures of any of the biologically active molecules or components comprising these molecules may be used.

### Compositions suitable for blocking DC-sign receptors

In another embodiment compositions suitable for the treatment and/or prevention of DC-sign mediated diseases, such as HIV or AIDS, are provided. Such compositions comprise a suitable amount of oligosaccharides, especially acid oligosaccharides as described hereinabove and in Example 1. Preferred are oligosaccharides which have a IC50 value of about 1000, 600, 400, more preferably 200µg/ml or less, such as 150, 100, 50, 25 µg/ml or less. The IC 50 value can be determined using methods known in the art (see Examples 1).

These compositions may additionally further comprise cysteine and/or source of cysteine, PUFAs, etc., as described elsewhere herein. The oligosaccharides may be formulated as a pharmaceutical composition or as a food or food supplement composition (as described herein below for compositions comprising oligosaccharides and cysteine and/or source of cysteine.

Guidance regarding pharmaceutical formulations that are suitable for various types of administration can be found in Remington's Pharmaceutical Sciences, Mace Publishing Company, Philadelphia, PA, 17th ed. (1985).

### Nutritional compositions and food supplements

It was found that the oligosaccharides and cysteine and/or source of cysteine can be advantageously applied in food, such as baby food and clinical food. Such food preferably comprises lipid, protein and carbohydrate and can be administered in a liquid or solid form. The term "liquid food" as used in the present invention includes dry food (e.g. powders) that are accompanied with instructions as to admix said dry food mixture with a suitable liquid (e.g. water). Solid food includes food in the form of a supplement bar with a water activity between 0.2 and 0.4. Water activity can be defined as the ratio of the water vapour pressure of a product to the vapour pressure of pure water at the same temperature. The solid product must meet target water activity otherwise the product will not be shelf stable. Also semi-solid food and food-supplements are provided.

Hence, the present invention also relates to a nutritional composition that in addition to the present oligosaccharides and cysteine and/or source of cysteine preferably comprises between 5 and 50 en% lipid, between 10 and 60 en% protein, between 15 and 85 en% carbohydrate. In the context of this ivention it is to be understood that the oligosaccharides in the compositions of the present ivention do not deliver calories and are therefore not included in the en% mentioned herein. All proteins, peptides, amino acids do contribute calories and therefore are included in the en% mentioned herein. In one embodiment the nutritional composition comprises between 15 and 50 en% lipid, between 25 and 60 en% protein and between 15 and 45 en% carbohydrate. In another embodiment the present nutritional composition comprises between 15 and 50 en% lipid, between 35 and 60 en% protein and between 15 and 45 en% carbohydrate.

Preferably lipids are used that have a high content of EPA or GLA. Fish oil and borage or evening primrose oil are preferred sources of these polyunsaturated fatty acids.

A source of digestible carbohydrate may be added to the nutritional formula. It preferably provides about 25% to about 40% of the energy of the nutritional composition. Any suitable (source of) carbohydrate may be used, for example sucrose, lactose, glucose, fructose, corn syrup solids, and maltodextrins, and mixtures thereof.

Preferably vitamins and minerals are present in amounts as required by FSMP regulations.

Diarrhea is a major problem in many HIV patients that receive liquid foods. It was found that stool problems are reduced by administering the present oligosaccharides in a dry nutritional composition or in liquid nutritional composition which have an osmolality between 50 and 500 mOsm/kg, more preferably between 100 and 400 mOsm/kg.

In view of the above, the nutritional composition preferably does not deliver excessive calories. Hence, the nutritional composition preferably does not contain more that 500 kcal/daily dose, more preferably between 200 and 400 kcal/daily dose and more preferably between 250 and 350 kcal/daily dose.

In accordance with the foregoing, the present invention relates to a nutritional composition comprising:
a) oligosaccharides, preferably the oligosaccharides comprise at least acid oligosaccharides preferably in such an amount that between 10 mg and 100 gram per day, preferably between about 100 mg and 50 grams per day, even more between about 0.5 and 20 gram is supplied in a daily dose, and
b) cysteine and/or source of cysteine, preferably wherein at least 0.1 g cysteine equivalent is supplied in a daily dose, and optionally
c) one or more biologically active ingredients (e.g. colostrum) and/or PUFA (e.g. EPA and/or GLA), preferably wherein at least 1 gram PUFA, more preferably between 1-50 gram PUFA, more preferably between 5 and 25 gram PUFA and even more preferably between 7.5 and 15 gram PUFA is supplied in a daily dose, also preferably at least 0.1 gram EPA and 0.05 gram GLA, more preferably between 0.1 and 5 gram EPA and between 0.05 and 2.5 gram GLA, more preferably between 0.5 and 2.5 gram EPA and between 0.25 and 1.25 gram GLA and even more preferably between 0.75 and 1.5 gram EPA and between 0.37 and 0.75 gram GLA is supplied in a daily dose.

In one embodiment the nutritional composition comprises between 5 and 50 en% lipid, between 35 and 60 en% protein, between 15 and 60 en% carbohydrate, acid oligosaccharides and cysteine and/or source of cysteine wherein the source of cysteine is selected from the group consisting of NAC, whey, colostrum, egg proteins or mixtures thereof.

In another embodiment the food composition comprises between 15 and 50 en% lipid, between 35 and 60 en% protein, between 15 and 45 en% carbohydrate, acid oligosaccharide and neutral oligosaccharide and cysteine or and/or source of cysteine wherein the source of cysteine is selected from the group consisting of NAC, colostrum, egg proteins or combinations thereof.

The nutritional composition is preferably in the form of or administered as a food supplement. This nutritional composition or food supplement can be advantageously used in a method for treating HIV patients, said method comprising administering said composition or supplement to a mammal, preferably a human infected with HIV .

Also provided is a method for manufacturing a composition for use in the treatment and/or prevention of HIV, said method comprising
- providing a suitable amount of one or more oligosaccharides;
- providing a suitable amount of cysteine and/or source of cysteine
- formulating both of the above components into a suitable food or food supplement or pharmaceutical composition.

The following examples illustrate the invention. Unless stated otherwise, the practice of the invention will employ standard conventional methods of molecular biology, pharmacology, immunology, virology, microbiology or biochemistry. Such techniques are described in Sambrook and Russell (2001) Molecular Cloning: A Laboratory Manual, Third Edition, Cold Spring Harbor Laboratory Press, NY, in Volumes 1 and 2 of Ausubel et al. (1994) Current Protocols in Molecular Biology, Current Protocols, USA and Remington's Pharmaceutical Sciences, Mack Publishing Company, Philadelphia, Pa., 17th ed. (1985), Microbiology: A Laboratory Manual (6th Edition) by James Cappuccino, Laboratory Methods in Food Microbiology (3rd edition) by W. Harrigan (Author) Academic Press, all incorporated herein by reference.

### EXAMPLES

### Example 1. Blockage of DC-SIGN - Fc binding by acid oligo's and GOS

Blocking DC-SIGN has been shown to prevent viral translocation from dendritic cells to CD4 T-cells. The inventors surprisingly found that oligosaccharides can block DC-SIGN with different efficacy. Acid oligosaccharides (AOS), like pectin hydrolysate, are the most potent as shown in Table 1. These results show that AOS can prevent binding of Fc fragments to DC-SIGN at the lowest concentration.

**TABLE 1. EFFICACY OF DC-sign BINDING BY OLIGOSACCHARIDES**

| **Oligosaccharide** | **I.C. 50 (µg/ml)** |
|---|---|
| Acid Oligosaccharide (pectin hydrolysate) | 200 |
| Galacto oligosaccharides (Trans galacto- oligosaccharides) | 600 |
| Fructooligosaccharide (Inuline HP) | >1000 |

### Material and methods:

Oligosaccharide preparations were coated on ELISA plate in serial dilutions. DC-SIGN - Fc binding was measured in an ELISA using anti-DC-SIGN- Fc and was visualized by adding a labeled secondary antibody. OD was measured with a spectrophotometer (Becton Dickinson) after 20 minutes of incubation. Results are depicted as the inhibitory concentration at 50% inhibition.

**Example 2. Composition of a nutritional bar**

| **Raw Material** | **Code** | **g/ day** | **protein** | **g/ 100g** |
|---|---|---|---|---|
| Colostrum | SR | 20.00 | 15.00 | 27.38 |
| borage oil (Ropufa 25 n-6) | 2000342 | 4.00 | 0.00 | 5.48 |
| EPA-DHA oil (Maruha) | 2001292 | 6.00 | 0.00 | 8.21 |
| Galacto-oligosaccharides Elix'or syrup | 2001189 | 15.38 | 0.00 | 21.06 |
| Inuline (Raftiline HP) | 2001190 | 0.79 | 0.00 | 1.08 |
| Acid Oligos (pectin hydrol.) | SR | 8.54 | 0.11 | 11.69 |
| N-acetyl-Cysteine | SR | 1.83 | 1.34 | 2.50 |
| Fructosestroop | JJ | 13.20 | 0.00 | 18.07 |
| Glycerine | JJ | 3.30 | 0.00 | 4.52 |

| | **per day kcal** | **En%** | | |
|---|---|---|---|---|
| energy protein | 66 | 26.9 | | |
| energy carbohydrates | 82 | 33.4 | | |
| energy fat | 97 | 39.7 | | |
| | *245* | | | |

**Example 3. Composition of a nutritional bar**

| **Raw Material** | **Code** | **g/ day** | **protein** | **carbs** | **fat** | **g/100g** |
|---|---|---|---|---|---|---|
| Colostrum | SR | 20.00 | 15.00 | 2.10 | 0.80 | 21.04 |
| borage olie (Ropufa 25 n-6) | 2000342 | 4.00 | 0.00 | 0.00 | 4.00 | 4.21 |
| EPA-DHA oil (Maruha) | 2001292 | 6.00 | 0.00 | 0.00 | 6.00 | 6.31 |
| Galacto-oligosaccharides (Elixer or syrup) | 2001189 | 15.38 | 0.00 | 4.78 | 0.00 | 16.18 |
| Inuline (Raftiline HP) | 2001190 | 0.79 | 0.00 | 0.00 | 0.00 | 0.83 |
| Acid Oligos (pectin hydrol.) | SR | 8.54 | 0.11 | 0.09 | 0.00 | 8.98 |
| Egg shell membrane powder | | 21.09 | 16.87 | 0.00 | 0.00 | 22.19 |
| Fructosestroop | JJ | 15.40 | 0.00 | 11.92 | 0.00 | 16.20 |
| glycerine | JJ | 3.85 | 0.00 | 3.83 | 0.00 | 4.05 |
| *SUM* | | *95.05* | *31.98* | *22.72* | *10.80* | *100.00* |

| | **per day kcal** | **En%** | **per 100g kcal** | | | |
|---|---|---|---|---|---|---|
| energy protein | 128 | 40.5 | 135 | | | |
| energy carbs | 91 | 28.8 | 96 | | | |
| energy fat | 97 | 30.8 | 102 | | | |
| *SUM* | *316* | | *332* | | | |

**Example 4. Powder composition**

| **Raw Material** | **Code** | **g/ day** | **protein** | **carbs** | **fat** | **g/ 100g** |
|---|---|---|---|---|---|---|
| Colostrum | SR | 20.00 | 15.00 | 2.10 | 0.80 | 29.39 |
| borage oil (Ropufa 25 n-6) | 2000342 | 4.00 | 0.00 | 0.00 | 4.00 | 5.88 |
| EPA-DHA oil (Maruha) | 2001292 | 6.00 | 0.00 | 0.00 | 6.00 | 8.82 |
| GOS/MD DE2 powder | 2001189 | 14.78 | 0.00 | 7.66 | 0.00 | 21.72 |
| Inuline (Raftiline HP) | 2001190 | 0.79 | 0.00 | 0.00 | 0.00 | 1.16 |
| Acid Oligos (pectin hydrol.) | SR | 8.54 | 0.11 | 0.09 | 0.00 | 12.55 |
| N-acetyl-Cysteine | SR | 1.83 | 1.34 | 0.00 | 0.00 | 2.69 |
| MD DE47 | MM | 7.00 | 0.01 | 6.75 | 0.02 | 10.29 |
| MD DE47 | MM | 5.00 | 0.01 | 4.82 | 0.01 | 7.35 |
| SSL (emulsifier) | SHS | 0.11 | 0.00 | 0.00 | 0.11 | 0.17 |
| *SUM* | | *68.05* | *16.46* | *21.41* | *10.94* | *100.0* |

| | **per day kcal** | **En%** | **per 100g kcal** | | | |
|---|---|---|---|---|---|---|
| energy protein | 66 | 26.3 | 97 | | | |
| energy carbs | 86 | 34.3 | 126 | | | |
| energy fat | 98 | 39.4 | 145 | | | |
| *SUM* | *250* | | *367* | | | |

**Example 5. Powder composition**

| **Raw Material** | **Code** | **g/ day** | **protein** | **carbs** | **fat** | **g/ 100g** |
|---|---|---|---|---|---|---|
| Colostrum | SR | 20.00 | 15.00 | 2.10 | 0.80 | 19.95 |
| borage oil (Ropufa 25 n-6) | 2000342 | 4.00 | 0.00 | 0.00 | 4.00 | 3.99 |
| EPA-DHA oil (Maruha) | 2001292 | 6.00 | 0.00 | 0.00 | 6.00 | 5.98 |
| GOS/MaltoDex (DE2 powder) | 2001189 | 14.78 | 0.00 | 7.66 | 0.00 | 14.74 |
| Inuline (Raftiline HP) | 2001190 | 0.79 | 0.00 | 0.00 | 0.00 | 0.79 |
| Acid Oligos (pectin hydrol.) | SR | 8.54 | 0.11 | 0.09 | 0.00 | 8.52 |
| alpha-lactalbumin (Davisco) | | 34.03 | 31.21 | 0.17 | 0.17 | 33.94 |
| MaltoDex DE47 | MM | 7.00 | 0.01 | 6.75 | 0.02 | 6.98 |
| MaltoDex DE47 | MM | 5.00 | 0.01 | 4.82 | 0.01 | 4.99 |
| SSL (emulsifier) | SHS | 0.11 | 0.00 | 0.00 | 0.11 | 0.11 |
| *SUM* | | *100.25* | *46.33* | *21.58* | *11.11* | *100.00* |

| | **per day kcal** | **En%** | **per 100g kcal** | | | |
|---|---|---|---|---|---|---|
| energy protein | 185 | 49.9 | 185 | | | |
| energy carbs | 86 | 23.2 | 86 | | | |
| energy fat | 100 | 26.9 | 100 | | | |
| *SUM* | *372* | | *371* | | | |

**Example 6. Liquid nutritional composition**

| **Raw Material** | **Code** | **g/ day** | **protein** | **carbs** | **fat** | **g/ ltr** |
|---|---|---|---|---|---|---|
| borage oil (Ropufa 25 n-6) | 2000342 | 4.00 | 0.00 | 0.00 | 4.00 | 10.67 |
| EPA-DHA oil (Maruha) | 2001292 | 6.00 | 0.00 | 0.00 | 6.00 | 16.00 |
| Galacto-oligosacchariden (Elixer or syrup) | 2001189 | 15.38 | 0.00 | 4.78 | 0.00 | 41.01 |
| Inuline (Raftiline HP) | 2001190 | 0.79 | 0.00 | 0.00 | 0.00 | 2.11 |
| Acid Oligosaccharides (pectin hydrolysate) | SR | 8.54 | 0.11 | 0.09 | 0.00 | 22.77 |
| Egg shell membrane powder | SR | 21.09 | 16.87 | 0.00 | 0.00 | 56.24 |
| WPH (cysteine peptide) | SR | | 0.00 | 0.00 | 0.00 | 0.00 |
| MaltoDextrin (DE47) | MM | 18.80 | 0.02 | 18.12 | 0.05 | 50.13 |
| *SUM* | | *74.60* | *17.00* | *22.99* | *10.05* | *198.93* |

| | **per day kcal** | **En%** | **per Itr kcal** | | | |
|---|---|---|---|---|---|---|
| energy protein | 68 | 27.2 | 181 | | | |
| energy carbs | 92 | 36.7 | 245 | | | |
| energy fat | 90 | 36.1 | 241 | | | |
| *SUM* | *250* | | *668* | | | |

**Example 7. Liquid nutritional composition**

| **Raw Material** | **Code** | **g/ day** | **protein** | **carbs** | **fat** | **g/ Itr** |
|---|---|---|---|---|---|---|
| borage olie Ropufa 25 n-6 | 2000342 | 4.00 | 0.00 | 0.00 | 4.00 | 10.67 |
| Maruha EPA-DHA oil | 2001292 | 6.00 | 0.00 | 0.00 | 6.00 | 16.00 |
| Galacto-oligosacchariden (Elixer or syrup) | 2001189 | 15.38 | 0.00 | 4.78 | 0.00 | 41.01 |
| Raftiline HP (Inuline) | 2001190 | 0.79 | 0.00 | 0.00 | 0.00 | 2.11 |
| AOS (pectin hydrolysate) | SR | 8.54 | 0.11 | 0.09 | 0.00 | 22.77 |
| WPH (cysteine peptide) | SR | 24.19 | 20.83 | 0.92 | 0.02 | 64.51 |
| MD DE47 | MM | 13.50 | 0.02 | 13.01 | 0.03 | 36.00 |
| *SUM* | | *72.40* | *20.95* | *18.80* | *10.06* | *193.07* |

| | **per day kcal** | **En%** | **per Itr kcal** | | | |
|---|---|---|---|---|---|---|
| energy protein | 84 | 33.6 | 223 | | | |
| energy carbs | 75 | 30.1 | 201 | | | |
| energy fat | 91 | 36.3 | 241 | | | |
| *SUM* | *250* | | *665* | | | |

## Claims

1. Use of one or more oligosaccharides and cysteine and/or source of cysteine in the manufacture of a composition for use in a method for the treatment and/or prevention of HIV or AIDS in a mammal, said method comprising administering to said mammal a composition comprising a therapeutically effective amount of oligosaccharides and cysteine and/or source of cysteine.

2. Use according to claim 1 wherein the cysteine and/or source of cysteine provide at least 100 mg cysteine equivalent in a daily dose.

3. Use according to claim 1 or 2 wherein the cysteine and/or source of cysteine provide at least 600, preferably at least 1000 mg, cysteine equivalent in a daily dose.

4. Use according to any of the preceding claims, wherein the oligosaccharides are acid oligosaccharides comprising at least one terminal uronic acid unit.

5. Use according to claim 4, wherein the uronic acid unit is selected from the group consisting of galacturonic acid, glucuronic acid, guluronic acid, iduronic acid, mannuronic acid, riburonic acid and alturonic acid.

6. Use according to any of the the preceding claims, wherein of the oligosaccharide the terminal hexose unit has the following structure: 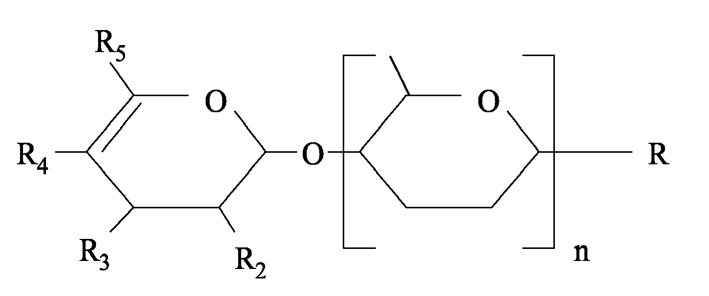 wherein;
R is selected from the group consisting of hydrogen, hydroxy or acid group; and at least one selected from the group consisting of R₂, R₃, R₄ and R₅ represent N-acetylneuraminic acid, N-glycoloylneuraminic acid, free or esterified carboxylic acid, sulfuric acid group and phosphoric acid group, and the remaining of R₂, R₃, R₄ and R₅ represent hydroxy and/or hydrogen; and n is an integer from 1-5000.

7. Use according to any of claims 4-6, wherein the terminal uronic acid has the structure as depicted in claim 6 and wherein R represents one selected from the group consisting of hydrogen, hydroxy or acid group ; and one selected from the group consisting of R₂, R₃, R₄ and R₅ represents free or esterified carboxylic acid; and the remaining of R₂, R₃, R₄ and R₅ represent hydroxy and/or hydrogen.

8. Use according to any one of the preceding claims, wherein the acid oligosaccharide has a degree of polymerisation between 1 and 250, preferably between 1 and 10.

9. Use according to any of the preceding claims, wherein the composition further comprises neutral oligosaccharides.

10. Use according to claim 9, wherein at least 15% of the total oligosaccharides comprise of acid oligosaccharides.

11. Use according to any of the preceding claims, wherein the source of cysteine is N-Acetyl cysteine, whey, colostrum, egg proteins or a combination thereof.

12. Use according to claim 11 wherein the whey, colostrums or egg proteins are at least partially hydrolyzed.

13. Use according to any of the preceding claims, wherein the composition further comprises polyunsaturated fatty acids (PUFA).

14. Use according to claim 13 wherein the PUFA comprises at least 20% GLA plus EPA, based on the total fatty acid content.

15. A food composition comprising between 15 and 50 en% lipid, between 25 and 60 en% protein, between 15 and 45 en% carbohydrate, acid oligosaccharide and neutral oligosaccharide and cysteine and/or source of cysteine wherein the cysteine and/or source of cysteine is selected from the group consisting of NAC, whey, colostrum, egg proteins or combinations thereof.

16. A food composition comprising between 15 and 50 en% lipid, between 35 and 60 en% protein, between 15 and 45 en% carbohydrate, acid oligosaccharide and neutral oligosaccharide and cysteine and/and/or source of cysteine wherein the and/or source of cysteine is selected from the group consisting of NAC, colostrum, egg proteins or combinations thereof.

17. A food composition comprising according to claims 15 or 16, wherein said lipid comprises EPA and/or GLA.
